(19) Europäisches Patentamt
European Patent Office
Office européen des brevets

(11) EP 1 400 508 B1

(12) EUROPÄISCHE PATENTSCHRIFT

(45) Veröffentlichungstag und Bekanntmachung des Hinweises auf die Patenterteilung:
**24.12.2014 Patentblatt 2014/52**

(21) Anmeldenummer: **03019662.0**

(22) Anmeldetag: **08.09.2003**

(51) Int Cl.:
***C07D 231/12*** (2006.01) ***C07D 233/54*** (2006.01)
***C07D 233/56*** (2006.01) ***C07D 249/08*** (2006.01)
***C07C 333/24*** (2006.01) ***C08F 2/38*** (2006.01)
***C08F 36/18*** (2006.01) ***C07D 207/325*** (2006.01)

(54) **Dithiocarbaminsäureester**

Dithiocarbamic acid ester

Esters d'acide dithiocarbamique

(84) Benannte Vertragsstaaten:
**DE FR**

(30) Priorität: **20.09.2002 DE 10243666**

(43) Veröffentlichungstag der Anmeldung:
**24.03.2004 Patentblatt 2004/13**

(73) Patentinhaber: **LANXESS Deutschland GmbH**
**50569 Köln (DE)**

(72) Erfinder:
- **Achten, Dirk, Dr.**
  **50823 Köln (DE)**
- **Klimpel, Michael, Dr.**
  **50269 Pulheim-Stommeln (DE)**
- **Barriau, Emilie**
  **55116 Mainz (DE)**
- **Reif, Lothar, Dr.**
  **41541 Dormagen (DE)**
- **Mottweiler, Renke, Dr.**
  **51373 Leverkusen (DE)**
- **Berg, Heinrich**
  **50259 Pulheim (DE)**
- **Szentivanyi, Zsolt, Dr.**
  **51373 Leverkusen (DE)**
- **Glander, Stefan, Dr.**
  **N7T 7H6 Sarnia**
  **Ontario (CA)**

(56) Entgegenhaltungen:
**EP-A- 0 421 149** **WO-A-99/31144**
**CH-A- 533 106** **DE-A- 2 131 135**
**US-A1- 2002 061 990**

- **PONGO, LASZLO ET AL: "On triazoles. XXXIII. The reaction of potassium triazolyldithiocarbonates with dihaloalkanes" JOURNAL OF HETEROCYCLIC CHEMISTRY , Bd. 31, Nr. 4, 1994, Seiten 997-1004, XP009022975**
- **DATABASE CROSSFIRE BEILSTEIN [Online] Beilstein Institut zur Förderung der Chemischen Wissenschaften, Frankfurt am Main, DE; Database accession no. BRN 1775086 XP002264572 & CHILDS ET AL.: J. CHEM. SOC., 1948, Seite 2182**
- **DATABASE CROSSFIRE BEILSTEIN [Online] Beilstein Institut zur Förderung der Chemischen Wissenschaften, Frankfurt am Main, DE; Database accession no. BRN 1372724 XP002264573 & SAKURAI ET AL.: SYNTHESIS, 1978, Seite 370**
- **DATABASE CA [Online] CHEMICAL ABSTRACTS SERVICE, COLUMBUS, OHIO, US; ABE, TETSUYA ET AL: "Preparation of difluoroalkenyl carbamates as pesticides" retrieved from STN Database accession no. 139:197278 CA XP002264574 & JP 2003 238518 A (KUMIAI CHEMICAL INDUSTRY CO., LTD., JAPAN;IHARA CHEMICAL INDUSTRY CO.,) 27. August 2003 (2003-08-27)**
- **SAVARIMUTHU PHILIP ANTHONY, SUNIL VARUGHESE AND SYLVIA M. DRAPER: "Switching and tuning organic solid-state luminescencevia a supramolecular approach", CHEMCOMM, 4 November 2009 (2009-11-04), pages 7500-7502,**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

- **GEMMA GARRIDO, EVE KOORT, CLARA RÀFOLS, ELISABETH BOSCH, TOOMAS RODIMA, IVO LEITO, AND MARTÍ ROSÉS: "Acid-Base Equilibria in Nonpolar Media. Absolute pKa Scale of Bases in Tetrahydrofuran", J. ORG. CHEM., vol. 71, 31 October 2006 (2006-10-31), pages 9062-9067,**
- **Akio Sakurai ET AL: "Conversion of Dithiocarbamates to Iodides via S -Alkylation", Synthesis, vol. 1978, no. 05, 1 January 1978 (1978-01-01), pages 370-372, XP055094533, ISSN: 0039-7881, DOI: 10.1055/s-1978-24749**


Bemerkungen:

Die Akte enthält technische Angaben, die nach dem Eingang der Anmeldung eingereicht wurden und die nicht in dieser Patentschrift enthalten sind.

## Beschreibung

Die vorliegende Erfindung betrifft bestimmte Dithiocarbaminsäureester, deren Herstellung und deren Verwendung zur Regelung des Polymerisationsgrades bei der Polymerisation von Monomeren, insbesondere bei der Polymerisation von Chloropren zu Polychloropren, und bei der Polymerisation von 2,3-Dichlorbutadien zu Poly-2,3-dichlorbutadien und bei der Copolymerisation von Chloropren mit 2,3-Dichlorbutadien. Weiterhin betrifft die vorliegende Erfindung Polymere, die durch die Polymerisation von Monomeren in Gegenwart der erfindungsgemäßen Dithiocarbaminsäureester erhältlich sind. Weiterhin betrifft die vorliegende Erfindung Polymere, die Endgruppen enthalten, die von den erfindungsgemäßen Dithiocarbaminsäureestern abgeleitet sind.

WO 99/31144 offenbart die Verwendung von Dithiocarbaminsäureestern zur Regelung des Polymerisationsgrades bei der Polymerisation von Vinyl-Monomeren.

DE-A 21 56 453 offenbart Dialkoxyxanthogendisulfide und ihre Verwendung als Regler bei der Polymerisation von Dienen.

Die Verwendung von Dithiocarbonsäureestern und Xanthogensäureestern zur Regelung der Polymerisation von Vinylmonomeren wird in WO 98/01478 für die Dithiocarbonsäureester und in WO 01/42312 und WO 98/58974 für die Xanthogensäureester beschrieben.

Auch WO 99/35177 und Tetrahedron Letters 1999, Seite 2435 ff. beschäftigen sich mit Polymerisationsreglern.

Ein Verfahren zur kontrollierten Darstellung von Polymeren auf Basis von Dienen in Gegenwart von Dithiocarbonsäureester, Xanthogensäureester, Dithiocarbaminsäureester, welches mit guten Ausbeuten in vertretbarer Zeit zu technisch relevanten Molekulargewichten Mn > 50.000 g/mol führt, ist nicht beschrieben, ist jedoch erstrebenswert.

Die Dithiocarbonsäureester/Xanthogensäureester/Dithiocarbaminsäureester, müssen grundsätzlich in guter Reinheit und in hoher Ausbeute technisch herstellbar sein, um in der betrieblichen Praxis zur Regelung des Polymerisationsgrades bei der Polymerisation von Vinylmonomeren und Dienmonomeren geeignet zu sein.

Verfahren zur Herstellung von Dithiocarbaminsäureestern sind bekannt und werden beispielsweise beschrieben in Houben-Weyl (Hrsg.: K. H. Bückel, J. Falbe, H. Hagemann, M. Hanack, B. Klamann, R. Kreher, H. Kropf, M. Regitz), Thieme Verlag, Stuttgart 1983, 4. Auflage, Band E 4, Seiten 458-478.

Beispielsweise offenbaren Journal of Heterocyclic Chemistry, Bd. 31, Nr. 4, 1994, Seiten 997-1004, CH 533 106 A, DE 21 31 135 A oder Synthesis, Bd. 1978 Nr.5, Seiten 370-372 Dithiocarbaminsäureester, allerdings jedoch keine Verbindungen, welche einen unsubstituierten heterocyclischen Substituenten tragen, der in protonierter Form einen pKs im Bereich zwischen 12 und 20 aufweist.

Dithiocarbaminsäureester können im Allgemeinen nach folgendem literaturbekanntem Verfahren hergestellt werden. Durch die Umsetzung von Aminen mit Schwefelkohlenstoff in Gegenwart von mindestens äquimolaren Mengen einer Base (z.B. Kaliumhydroxid) in wässriger Lösung stellt man Dithiocarbamate her, welche nach Isolierung durch Umsetzung mit einer organischen Halogenverbindung unter Abspaltung des Salzes aus der Base und dem Halogenid zu Dithiocarbaminsäureestern umgesetzt werden können.

WO 99/31144 offenbart die Verwendung von Natriumhydrid in organischen Solventien als Deprotonierungsreagenz.

Für eine technische Verwendung sind Dithiocarbaminsäureester, dargestellt nach den Synthesevorschriften in WO 99/31144, wenig geeignet aufgrund zu geringer Ausbeuten und nicht ausreichender Produktselektivität. Die erhaltenen Reinheiten sind ohne Zwischenschaltung eines Reinigungsschrittes für den technischen Einsatz dieser Dithiocarbaminsäureester als Regler (d.h. zur Regelung des Polymerisationsgrades bei der Polymerisation von Monomeren) nicht ausreichend. WO 99/31144 offenbart keine konkreten Dithiocarbaminsäureester enthaltend einen halogensubstituierten Alkylrest.

Die wie in WO 99/31144 beschrieben hergestellten Dithiocarbaminsäureester lassen sich gegebenenfalls durch Destillation, Umkristallisation oder Chromatographie so rein erhalten, dass ihr Einsatz als Polymerisationsregler möglich wird. Eine hohe Reinheit der Dithiocarbaminsäureester aufgrund der analytisch identifizierten, zum Teil mercaptanischen Nebenprodukte (stark riechend), welche selber regelnde Wirkung in der Polymerisation von Vinylmonomeren und Dienen zeigen, ist geboten.

Eine Destillation ist aufgrund der inhärenten thermischen Instabilität der Dithiocarbaminsäureester verlustreich und kann wiederum zu den genannten Nebenprodukten führen. Ebenso ist eine Reinigung durch Kristallisation mit bedeutenden Ausbeuteverlusten verbunden, während eine chromatographische Reinigung im technischen Maßstab nicht ausreichend kostengünstig realisierbar ist.

Ein Verfahren, welches geeignete Dithiocarbaminsäureester auf Basis billiger Ausgangschemikalien in guter Reinheit herzustellen gestattet, wäre somit ein bedeutender technischer Fortschritt.

Um die Reinheit der nach dem patentgemäßen Verfahren hergestellten Dithiocarbaminsäureester zu ermitteln, wurde außer den üblichen physikalischen Daten ihre Eignung als Molekulargewichtsregler bei der Emulsionspolymerisation von Chloropren getestet. Dabei wurde Chloropren in einem standardisierten Verfahren unter Zusatz kontrollierter Mengen von Dithiocarbaminsäureester polymerisiert. Das resultierende Elastomer (Polychloropren) wurde aufgearbeitet. Von dem so erhaltenen Produkt wurde die Viskosität als Lösungsviskosität aus dem Feststoff (5 %-ige Lösung in Toluol) bzw. Latex (8,6 %-ige Lösung in Toluol) mit Hilfe eines Brookfieldviskosimeters bei 20 °C ermittelt. Das Molekulargewicht Mn (die mittlere molare Masse) wurde mittels GPC (Gelpermeationschromatographie) mit Bezug auf eine Polystyroleichung bestimmt.

Chloropren, das zu Polychloropren polymerisiert werden kann, hat folgende Struktur:

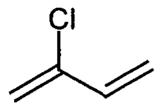

Gängige Regler für die Polymerisation von Chloropren wie beispielsweise Dodecylmercaptan oder Xanthogendisulfide erbringen Produkte mit nur mäßiger Molekulargewichtskontrolle. Die erhaltenen Molekulargewichte (zum Beispiel bestimmt anhand der Mooneyviskosität nach ISO 289 ohne Vorbehandlung) sind abhängig von eingesetzter Reglermenge, Teilchengröße, Reglerdiffusion sowie Zahl aktiver Ketten in einer Mizelle, im Falle einer Emulsionspolymerisation. Eine Einstellung des Molekulargewichtes über den Umsatz ist somit nicht möglich.

Die Herstellung von Polychloropren aus Chloropren ist bekannt. Sie wird üblicherweise als Emulsionspolymerisation durchgeführt. Das Verfahren der Emulsionspolymerisation verläuft zweistufig, wobei in der ersten Stufe die Polymerisation zum Latex durchgeführt wird und in der zweiten Stufe die Aufarbeitung des Latex zum Festkautschuk, beispielsweise durch Gefrierkoagulation, erfolgt. Das Verfahren setzt ein Molekulargewicht des Produktes von > 20 Mooney-Einheiten (siehe ISO 289) voraus, um eine Verarbeitbarkeit nach den oben erwähnten Verfahren zu gewährleisten. Dies entspricht etwa einem Zahlenmittel des Molekulargewichts (Mn) nach GPC (Polystyroleichung) von etwa>100.000g/mol.

Der vorliegenden Erfindung liegt daher die Aufgabe zugrunde, eine Verbindung bereitzustellen, die zur Regelung des Molekulargewichtes bei der Polymerisation eines oder mehrerer verschiedener Monomere geeignet ist, wobei mindestens ein Monomer eine Diengruppe enthält.

Weiterhin liegt der vorliegenden Erfindung die Aufgabe zugrunde, ein Verfahren zur Herstellung dieser Verbindung bereitzustellen sowie ein Verfahren zur Polymerisation von Monomeren in Gegenwart dieser Verbindung bereitzustellen.

Schließlich liegt der vorliegenden Erfindung die Aufgabe zugrunde, Polymere bereitzustellen, die durch Polymerisation in Gegenwart der genannten Verbindung erhältlich sind.

Diese Aufgabe wird gelöst durch eine Verbindung der Formel (I),

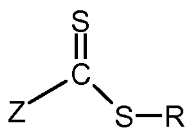

(I)

worin

R ein halogensubstituierter Alkenylrest, bevorzugt ein Rest der Formel

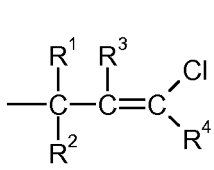

ist, in der $R^1$, $R^2$, $R^3$ und $R^4$ unabhängig voneinander H oder $C_1$-Alkyl bis $C_4$-Alkyl sind, wobei die cis-Form und die trans-Form gleichermaßen bevorzugt sind, besonders bevorzugt 3-Chlor-2-butenyl ist, und worin

Z entweder ein substituierter oder unsubstituierter heterocyclischer Rest ist, der mindestens ein Stickstoffatom enthält und der an einem Stickstoffatom an die - $CS_2$-R Gruppe aus Formel (I) angebunden ist und der in der zugrunde liegenden Form, in der an dem Stickstoffatom, an dem die Anbindung an die -$CS_2$-R Gruppe aus Formel (I) erfolgt, ein Wasserstoffatom gebunden ist, einen $pK_s$-Wert im Bereich zwischen 12 und 20 hat, vorzugsweise zwischen 14 und 18,

und wobei Z eine der folgenden Bedeutungen hat:

Pyrrol ($pK_s$-Wert = 17), Imidazol ($pK_s$-Wert = 14,5), Pyrazol ($pK_s$-Wert = 14,0), Indol ($pK_s$-Wert = 17), Carbazol ($pK_s$-Wert = 17), 2-Piperidinon ($pK_s$-Wert = 16,6), 2-Azepanon ($pK_s$-Wert = 16,6), 2-Azocanon ($pK_s$-Wert = 16,6).

Weiterhin wird die erfindungsgemäße Aufgabe gelöst durch ein Verfahren zur Herstellung der Verbindung der Formel (I) umfassend

a) das Bereitstellen einer Verbindung der Formel (IV),

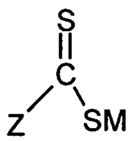

(IV)

wobei Z die genannte Bedeutung hat und M ein Alkalimetall ist, vorzugsweise Kalium,

b) das Umsetzen dieser Verbindung mit einer Verbindung der Formel (V),

R-X (V)

wobei R die für Formel (I) genannte Bedeutung hat und X für Cl, Br oder I steht, bevorzugt Cl oder Br.

Die Verbindung nach Formel (I) und das Verfahren zu ihrer Herstellung sind Gegenstand der vorliegenden Erfindung.

Weiterhin ist Gegenstand der vorliegenden Erfindung die Verwendung der Verbindung der Formel (I) wie oben definiert zur Regelung des Polymerisationsgrades bei der Polymerisation von Monomeren.

Weiterhin ist Gegenstand der vorliegenden Erfindung ein Verfahren zur Herstellung eines Polymeren durch Polymerisation von Monomeren in Gegenwart der Verbindung der Formel (I) wie oben definiert.

Das Folgende gilt für die genannte Verwendung und für das genannte Verfahren.

Es kann ein Monomer oder mehrere verschiedene Monomere polymerisiert werden. Wenn mehrere verschiedene Monomere polymerisiert werden, dann spricht man von Copolymerisation.

In einer besonderen Ausführungsform werden eines oder mehrere verschiedene Monomere polymerisiert, wobei alle Monomere entweder eine Vinylgruppe enthalten oder eine Diengruppe enthalten, und wobei mindestens ein Monomer eine Diengruppe enthält. Dabei ist in einer besonderen Ausführungsform ein Monomer Chloropren und / oder 2,3-Dichlorbutadien.

In einer besonderen Ausführungsform wird das Verfahren zur Herstellung eines Polymeren in Emulsion durchgeführt (sogenannte Emulsionspolymerisation).

Weiterhin ist Gegenstand der vorliegenden Erfindung ein Polymer erhältlich nach diesem Verfahren.

Weiterhin ist Gegenstand der vorliegenden Erfindung ein Polymer, das Endgruppen der Formel (VI)

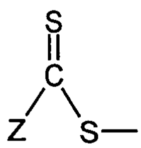

(VI)

und Endgruppen der Formel (VI a)

R- (VIa)

enthält, wobei Z und R die oben für Formel (I) definierte Bedeutung haben.

In einer besonderen Ausführungsform enthält das Polymer Wiederholungseinheiten abgeleitet von einem Monomer, das Diengruppen enthält, insbesondere abgeleitet von Chloropren und / oder 2,3-Dichlorbutadien. In einer weiteren besonderen Ausführungsform enthält das Polymer zusätzlich Wiederholungseinheiten abgeleitet von einem oder mehreren verschiedenen Monomeren, die eine Vinylgruppe enthalten.

Die vorliegende Erfindung hat unter anderem den Vorteil, eine Verbindung bereitzustellen, die zur Regelung

des Molekulargewichtes bei der Polymerisation von Monomeren geeignet ist, wobei zumindest ein Monomer oder alle eine Diengruppe enthalten, (bevorzugt Chloropren), wobei eine Abhängigkeit zwischen dem Molekulargewicht und der Reglermenge sowie dem Umsatz des Monomeren besteht.

Weiterhin hat sie den Vorteil, ein effizientes, technisch umsetzbares Verfahren zur Reindarstellung dieser Verbindung bereitzustellen.

Weiterhin hat sie den Vorteil, ein neues, im Vergleich zu gängigen Emulsionspolymerisationen verbessertes Verfahren zur Polymerisation von Monomeren bereitzustellen, wobei zumindest ein Monomer oder alle eine Diengruppe enthalten, (bevorzugt Chloropren). Das neue Verfahren zeichnet sich durch eine verbesserte Kontrolle von Molekulargewicht und Molekular-gewichtsverteilung ohne Verlust an Polymerisationsgeschwindigkeit aus und führt damit gegenüber herkömmlichen Verfahren zu höheren Durchsatzraten und einer verminderten Anzahl von Fehlproduktionen bei gleichzeitig höherer Produktqualität.

Weiterhin hat sie den Vorteil, Polymere bereitzustellen, die durch Polymerisation in Gegenwart der genannten Verbindungen erhältlich sind. Die erhaltenen Polymere zeichnen sich durch eine enge Molekulargewichtsverteilung, wenn gewollt aber auch verfahrenstechnisch eingestellt gezielt breite oder bimodale Verteilung, sowie durch definierte Endgruppen aus. Eng verteilte Polymere auf Basis von Monomeren, wobei zumindest ein Monomer oder alle eine Diengruppe enthalten (bevorzugt Chloropren), die klassisch durch Emulsionspolymerisation nicht zugänglich sind, zeichnen sich bei gummitechnologischen Prüfungen durch verbesserte Vulkanisatfestigkeiten gegenüber breitverteilten Polymeren gleichen mittleren Molekulargewichtes aus. Bimodale Polymere sind im Allgemeinen kautschuktechnologisch besser verarbeitbar gegenüber unimodalen Polymeren gleichen mittleren Molekulargewichtes.

Verbindungen der Formel (I)

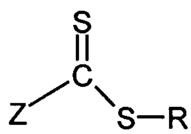

(I)

gehören zur Gruppe der Dithiocarbaminsäureester.

Als erfindungsgemäße Dithiocarbaminsäureester der Formel (I) seien beispielhaft genannt (sowohl die cis-Form als auch die trans-Form sind erfindungsgemäß)

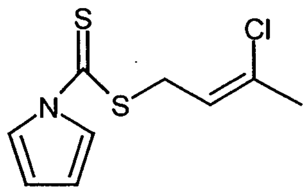

1)

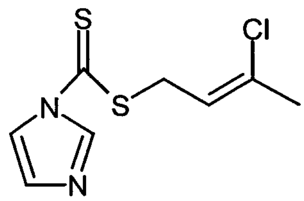

2)

1) 3-Chloro-2-butenyl-*1H*-pyrrol-1-carbodithioat
2) 3-Chloro-2-butenyl-*1H*-imidazol-1-carbodithioat

Eine Ausführungsform des erfindungsgemäßen Verfahrens zur Herstellung der Verbindung nach Formel (I) ist dadurch gekennzeichnet, dass man die Reaktion ohne Aufarbeitungsstufe oder Reinigungsstufe in gleicher organischer Phase durchführt, bevorzugt Ether, besonders bevorzugt Tetrahydrofuran, bei einer Temperatur von - 78 C bis 80 C, bevorzugt 0 bis 30 C zur Vermeidung von Nebenreaktionen, die zur Ausbildung von stark riechenden Nebenprodukten führen und eine weitere Reinigungsstufe notwendig machen. Das erhaltene Produkt wird nach Abfiltrieren des entstandenen Alkalihalogenids (z.B. Kaliumhydroxid) durch Eindampfen der Lösung rein erhalten.

In einem ersten Schritt wird das Carbaminsäuresalz Z-C(S)-S-M, wobei M bevorzugt Kalium ist, hergestellt durch Umsetzung von Z-H mit M in organischer Phase bei anschließender Zugabe von Kohlenstoffdisulfid.

Das Carbaminsäuresalz Z-C(S)-S-M wird isoliert oder in einer bevorzugten Form des Verfahrens in situ umgesetzt mit einer Verbindung R-X, wobei

R bevorzugt = 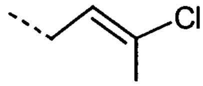

und
X bevorzugt Cl ist.

Die bevorzugte Ausgangsverbindung R-X ist als 1,3-Dichlorbuten-2 ein bekanntes Zwischenprodukt der technischen 2,3- Dichlorbutadiensynthese.

Die erfindungsgemäßen Verbindungen nach Formel (I) eignen sich besonders für die Regelung der Molmasse bei der Herstellung von Polychloropren und Polydichlorbutadien sowie deren Copolymeren. Polychloropren findet Verwendung als Kautschuk in der Gummiindustrie, sowie als Klebstoffrohstoff, sowie als Polychloroprenlatices, sowie auch in Abmischung mit vernetzten Chloroprenpolymeren.

Bei dem Verfahren zur Herstellung der Verbindung nach Formel (I) erfolgt die Deprotonierung der Amine bevorzugt in aprotischen polaren organischen Lösungsmitteln, eventuell in Gemischen von organischen Lösungsmitteln mit diesen, bevorzugt in Ethern, besonders bevorzugt in Tetrahydrofuran.

Erfindungsgemäß bevorzugte Monomere sind Chloropren, und 2,3-Dichlorbutadien.

Die erfindungsgemäßen Dithiocarbaminsäureester zeichnen sich durch eine deutlich bessere Reglerwirksamkeit im Vergleich zu klassischen Molekulargewichtsreglern auf Xanthogendisulfid- und Mercaptan-Basis aus und führen daher zu Polychloroprenlatices mit besserer Lagerstabilität. Darüber hinaus ist eine gezielte Ansteuerung des Molekulargewichts über den Umsatz sowie die eingesetzte Reglermenge möglich, wie dies bisher für Emulsionspolymerisate nicht möglich war.

2-Chlorbutadien (Chloropren) kann in wässriger Emulsion in Gegenwart von radikalischen Initiatoren polymerisiert werden. Es ist auch möglich, Chloropren mit verschiedenen Comonomeren zu polymerisieren. Gebräuchliche Comonomere sind z.B.: 1-Chlorbutadien, 2,3-Dichlorbutadien, Styrol, Divinylbenzol, Ethylenglycoldi (meth)acrylat, Isopren, Acrylnitril, Acrylate und Methacrylate.

Zum Beispiel kann durch Zusatz von 3-Chloro-2-butenyl-*1H*-pyrrol-1-carbodithioat (1), als Beispiel eines erfindungsgemäßen Reglers, das Molekulargewicht des im Rahmen einer Emulsionspolymerisation entstandenen Polymeren gesteuert werden. Die Polymerisationstemperatur liegt üblicherweise zwischen 5 und 80°C, bevorzugt bei 10 bis 50°C. Bei diesen Reaktionstemperaturen wird die Polymerisation üblicherweise bei einem Monomerumsatz von 50 bis 90 %, insbesondere 60 bis 80 %, abgebrochen. Als geeignete Emulgatorsysteme werden üblicherweise Alkalisalze wasserlöslicher gesättigter oder ungesättigter Monocarbonsäuren eingesetzt, z.B. (gegebenenfalls disproportionierte) Resinsäuren, gegebenenfalls im Gemisch mit Fettsäuren wie Ölsäure oder Kokosfettsäuren. Die Emulgatoren werden üblicherweise in Mengen von 2 -10 Gew.-Teilen (bevorzugt 3 bis 5 Gew.-Teile), bezogen auf 100 Teile Monomer, zugesetzt. Auch Kondensationsprodukte aus Naphthalinsulfonsäure und Formaldehyd können als zusätzliche Emulgatoren eingesetzt werden.

Die Polymerisation wird üblicherweise durch Zugabe von bekannten Polymerisationsinitiatoren gestartet und durchgeführt. Als Initiatoren kommen insbesondere Radikale erzeugende Verbindungen in Frage, zum Beispiel Alkalipersulfate, Wasserstoffperoxid und organische Peroxide wie Benzoylperoxid, Cumolhydroperoxid oder Redoxinitiatoren wie Kaliumperoxodisulfat / Natriumdithionit / Natriumsulfit, Kaliumperoxodisulfat / Antrachinon-2-sulfonsäurenatriumsalz.

In einem bevorzugten Verfahren wird die Polymerisation durch Zugabe von Initiatoren, die bei niedrigen Temperaturen thermisch zerfallen, wie Formamidinsulfinsäure, initiiert. Inhibitoren wie Phenothiazin und Diethylhydroxylamin können die Polymerisation beenden.

Das restliche nicht umgesetzte Monomere kann durch Wasserdampfdestillation entfernt werden. Der pH des alkalischen Latex kann durch verdünnte Essigsäure auf pH 5-7 gesenkt und das Polymere aus dieser Emulsion, beispielsweise durch Gefrierkoagulation, isoliert und getrocknet werden. Für die Aufarbeitung eignen sich aber auch andere herkömmliche Methoden, wie z.B. in der deutschen Patentschrift DE-A1111804 beschrieben.

Für die Herstellung von Klebstoffen kann das Polychloropren in organischen Lösungsmitteln wie Benzol, Toluol, Methylenchlorid oder Trichlorethylen bzw. in Gemischen dieser Lösungsmittel mit anderen Lösungsmitteln, die Polychloropren allein nicht lösen, wie Benzin, Cyclohexan oder Methylacetat, gelöst werden.

Die Viskosität der Lösung richtet sich nach dem Verwendungszweck und liegt vorzugsweise bei 10-100 Poise, gemessen bei 20 C mit einem Brookfield-LVT-Viskosimeter.

Weitere Methoden, Polychloroprenklebstoffe herzustellen, werden in DE-A 12 00 988 beschrieben.

Aliphatische Dithiocarbaminsäureester ausgehend von sekundären Aminen mit einem pKs-Wert unter 12 sind als Regler nicht geeignet. Die unter Benutzung dieser Dithiocarbaminsäureester erhaltenen Produkte zeigen sehr hohe Molekulargewichte in Folge mangelnder Reglerwirksamkeit. Der Einsatz der erfindungsgemäßen Dithiocarbaminsäureestern ausgehend von sekundären Aminen mit einem pKs von 12-20, bevorzugt 14-18, als Regler für die Polymerisation von Chloropren ist bislang nicht beschrieben.

## Beispiele

In Tabelle 1 sind der Umsatz und die Reglermenge sowie das erhaltene Molekulargewicht nach GPC des Polychloroprens aufgetragen, welches nach dem erfindungsgemäßen Verfahren (siehe unten) dargestellt wurde. Jeder

Eintrag entspricht dem Ergebnis eines Polymerisationsversuchs von Chloropren mit einer festgelegten Kombination von Regler und Umsatz. Man sieht, dass nur mit den erfindungsgemäßen Dithiocarbaminsäureestern eine gleichzeitige Kontrolle des Molekulargewichts über Umsatz und Reglermenge, im Idealfall nach folgender einfacher aus der lebenden ionischen Polymerisation bekannten Gleichung möglich ist.

Gleichung 1:

Molekulargewichtsmittel = (molare Menge Monomer·Molekulargewicht Monomer·Umsatz)/(molare Menge Regler)

Beispiele zum patentgemäßen Verfahren zur Emulsionspolymerisation von Chloropren:

In einem 3 1 Glasreaktor wurden als wässrige Phase vorgelegt (alle Teile sind Gewichtsteile): 125 Teile deionisiertes Wasser (1.250 g); 2,80 Teile Dresinate 731 als 70 %-ige Lösung (40 g); 0,3 Teile kondensierte Naphthalinsulfonsäure als 30 %-ige Lösung (10 g), 0,65 Teile NaOH (6,5 g). Dazu gab man eine Monomerphase bestehend aus 100 Teilen Chloropren (1000 g) und der gewünschten Menge X an Teilen Regler (siehe Tabelle 1). Vor Beginn der Polymerisation wurde der Reaktor 1,5 h mit Stickstoff gespült. Die Reaktion fand unter Stickstoffatmosphäre statt. Die Polymerisationszeit bis zum Erreichen von 60 % Umsatz betrug zwischen 1-5 h.

Variante A: Zur Initiierung wurde eine 2,5 %-ige Lösung von Formamidinsulfinsäure (FAS) in Wasser kontinuierlich hinzugefügt. Die Reaktionstemperatur betrug 45 C.

Variante B: Im Falle einer Redoxaktivierung wurden eine 1,5 %-ige Kaliumperoxodisulfat-Lösung (KPS) sowie eine 1,0 %-ige Natriumdithionit-Lösung (NHS) in Wasser kontinuierlich hinzugefügt. Die Reaktionstemperatur 10 °C.

Bei einem Monomerumsatz von 60 % wurde die Reaktion durch Zugabe von Phenothiazin abgebrochen. Das restliche Monomere wurde durch Wasserdampfdestillation aus dem Ansatz entfernt. Nach Absenken des pH-Wertes auf 7 wurde das Polymere mit 0,5 %-iger Magnesiumchloridlösung ausgefällt und die wässrige Phase abfiltriert. Das Produkt wurde gewaschen und im Vakuum über Nacht bei 50°C getrocknet.

Abkürzungen:

Dresinate 731 = Na-Salz der disproportionierten Resinsäure (im Handel erhältlich z.B. von der Firma Abieta) (Resinsäure ist ein Naturprodukt, vergleichbar mit Baumharz, und wird deshalb auch als Harzsäure bezeichnet)

Tabelle 1 gibt die Ergebnisse von Emulsionspolymerisationen von Chloropren mit verschiedenen Reglern nach Verfahrensvariante A wieder: Spalte 1 und 2 zeigen die Substituenten Z und R der Regler-Verbindung. Spalte 3 gibt die benutzte Methode wieder. In der vierten Spalte ist der pKs-Wert des protonierten Substituenten Z gemäß Formel (I) angegeben. Spalte 5 und 6 nennen Namen und Strukturformeln des verwendeten Reglers. Spalte 7 listet die Nummern der Regler-Verbindungen auf. Spalte 8 zeigt die Lösungsviskosität des erhaltenen Polymeren bei 60 % als Lösungsviskosität aus einer 8,6 % toluolischen Lösung gemessen. Spalte 9 nennt die Menge Regler in mmol, die dem Ansatz in der Monomerphase zugesetzt wurden. Spalte 10 nennt das erreichte Zahlenmittel des Molekulargewichtes nach GPC Messung. Spalte 11 differenziert zwischen erfindungsgemäßen Beispielen (Ziffern) und Vergleichsbeispielen (Buchstaben). Außer Regler-Verbindung (7) sind alle Vergleichsverbindungen nach Methode A durchgeführt worden. Im Fall von Regler-Verbindungen 8 bis 11 wurde ein Alkohol (Isopropylalkohol) anstatt eines Amins (Pyroll/Imidazol) verwendet.

**Tabelle 1**

| Z-H | R-X | Variante | pKs Wert Z-H | Name: nach IUPAC | Formel | Regler-Verbindung | Lösungsviskosität / mPas nach LVL | Teile Regler | $M_n$ | Beispiele/ Vergleichsbeispiele |
|---|---|---|---|---|---|---|---|---|---|---|
| 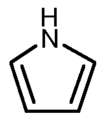 | 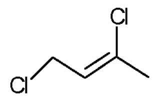 | A | 17.0 | 3-Chloro-2-butenyl 1H-pyrrole-1-carbodithioat (1) | 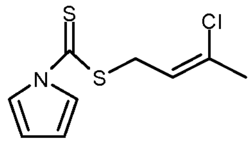 | (1) | 14 | 15 mmol | 50.000 | 1 |
| 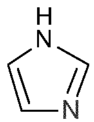 | 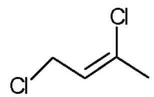 | A | 14.5 | 3-Chloro-2-butenyl 1 H-imidazole-1-carbodithioat (2) | 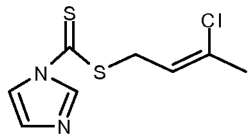 | (2) | 35 | 15 mmol | 101.000 | 2 |
| 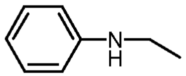 | 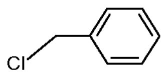 | A | 5.1 | Benzyl ethyl(phenyl)-dithiocarbamat (3) | 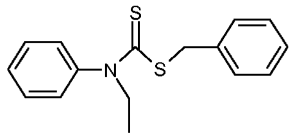 | (3) | vergelt | 15 mmol | | A |
| 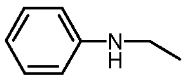 | 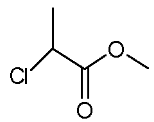 | A | 5.1 | Methyl 2-({[ethyl-(phenyl)amino] carbonothioyl}sulfanyl)propanoat (4) | 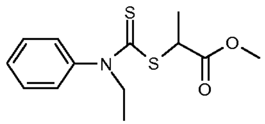 | (4) | vergelt | 15 mmol | | B |

(fortgesetzt)

| Z-H | R-X | Variante | pKs Wert Z-H | Name: nach IUPAC | Formel | Regler-Verbind-ung | Lösungsvisko-sität / mPas nach LVL | Teile Regler | $M_n$ | Beispiele/ Ver-gleichsbeispiele |
|---|---|---|---|---|---|---|---|---|---|---|
| 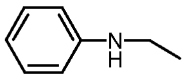 | 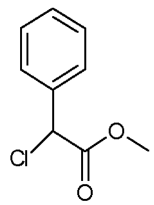 | A | 5.1 | Methyl ({[ethyl(phenyl)amino]carbonothioyl}sulfanyl)(phenyl)-acetat (5) | 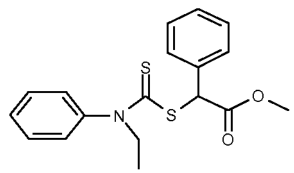 | (5) | vergelt | 15 mmol | | C |
| 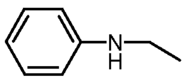 | 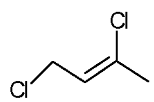 | A | 5.1 | [ethyl(phenyl)amino]carbonothioyl}sulfanyl-3 -chloro-2-butenyl (6) | 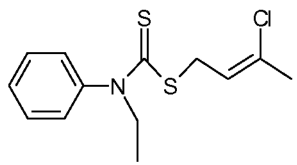 | (6) | vergelt | 15 mmol | | D |
| siehe DE-A 3 044 811 | | A | | Methylentrimethylolpropanxanthogendisulfid (7) | 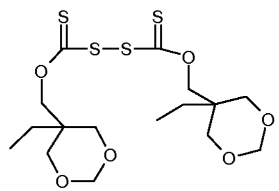 | (7) | 67 | 15 mmol | 168.000 | E |
| 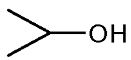 | 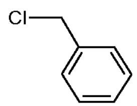 | A | | S-benzyl-O-isopropyl-dithiocarbonat (8) | 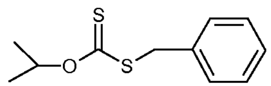 | (8) | vergelt | 15 mmol | | F |

(fortgesetzt)

| Z-H | R-X | Variante | pKs Wert Z-H | Name: nach IUPAC | Formel | Regler-Verbind-ung | Lösungsvisko-sität / mPas nach LVL | Teile Regler | $M_n$ | Beispiele/ Vergleichsbeispiele |
|---|---|---|---|---|---|---|---|---|---|---|
| 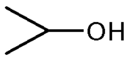 | 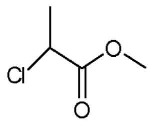 | A | | Methyl-2-[(iso-prop-oxy-carbono-thioyl) sulfanyl]propanoat (9) | 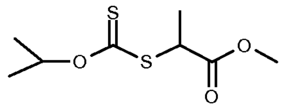 | (9) | vergelt | 15 mmol | | G |
| 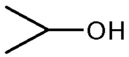 | 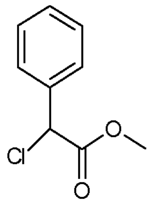 | A | | Methyl [(isopropoxycarbono-thioyl)sulfanyl] (phenyl)acetat (10) | 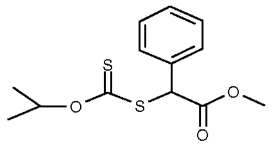 | (10) | vergelt | 15 mmol | | H |
| 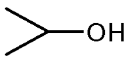 | 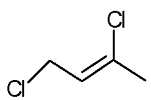 | A | | [(isopropoxycarbono-thioyl)sulfanyl] -3-chloro-2-butenyl (11) | 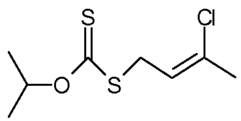 | (11) | vergelt | 15 mmol | | 1 |

Tabelle 2 beinhaltet Versuche zur Untersuchung des Molekulargewichts in Abhängigkeit von Umsatz und Verhältnis Monomermenge zu Reglermenge. Verglichen werden die Ergebnisse einer Emulsionspolymerisation von Chloropren mit einem Xanthogendisulfidregler (Regler-Verbindung (7) siehe Tabelle 1 und DE-A 304 48 11) und dem erfindungsgemäßen Dithiocarbaminsäureester (Regler-Verbindung (1) siehe Tabelle 1) hergestellt nach erfindungsgemäßem Verfahren und polymerisiert nach Variante A. In Spalte 1 wird die verwendete Reglerverbindung aufgeführt. Spalte 2 beinhaltet die eingesetzte Reglermenge. Spalte 3 enthält die Umsätze, die daraus nach Gleichung 1 berechneten theoretischen Molekulargewichte enthält Spalte 4. Spalte 5 und 6 beinhalten die experimentell ermittelten Molekulargewichte nach GPC und den Polydispersitätsindex' des Polymeren berechnet als den Quotienten aus dem Gewichtsmittel und dem Zahlenmittel nach GPC. Die Polymerisationen wurden bis zu einem Umsatz von 60 % geführt. Spalte 7 differenziert zwischen erfindungsgemäßen Beispielen (Ziffern) und Vergleichsbeispielen (Buchstaben).

**Tabelle 2**

| Regler-Verbindung | Teile Regler | Umsatz / % | Mn theor. | Mn (GPC) | Mw / Mn | Beispiel / Vergl.-Bsp. |
|---|---|---|---|---|---|---|
| (1) | 0,35 | 19<br>43<br>60 | 13.000<br>29.000<br>40.000 | 14.000<br>36.000<br>49.000 | 2,4<br>1,5<br>1,5 | 3 |
| (1) | 0,233 | 21<br>41<br>61 | 21.000<br>41.000<br>61.000 | 33.500<br>53.600<br>72.800 | 1,7<br>1,7<br>2,5 | 4 |
| (1) | 0,116 | 20,<br>39<br>60 | 40.000<br>78.000<br>120.000 | 56.500<br>84.600<br>131.800 | 1,6<br>1,6<br>1,8 | 5 |
| (1) | 0,075 | 25<br>41<br>61 | 78.000<br>127.000<br>190.000 | 102.000<br>127.000<br>190.000 | 1,8<br>1,8<br>2,3 | 6 |
| (7) | 0,75 | 20<br>40<br>60 | 34.000<br>34.000<br>34.000 | 148.700<br>142.800<br>148.700 | 2,1<br>2,3<br>2,6 | J |
| Mn = Zahlenmittel des Molekulargewichts<br>Mw = Gewichtsmittel des Molekulargewichts<br>GPC = Gel-Permeation-Chromatographie | | | | | | |

Tabelle 3 zeigt weitere Ergebnisse von Polymerisationen nach dem beschriebenen Verfahren, Variante A und B-auf. Verglichen werden verschiedene nach Tabelle 1 geeignete Dithiocarbaminsäureester-Regler in der Emulsionspolymerisation von Chloropren. In Spalte 1 wird die verwendete Reglerverbindung aufgeführt. Spalte 2 beschreibt die Variante der Verfahrensdurchführung. Spalte 3 beinhaltet die verwendete Reglermenge und die daraus nach Gleichung 1 berechneten theoretischen Molekulargewichte. Spalte 4 und 5 beinhalten die experimentell ermittelten Molekulargewichte nach GPC und den Polydispersitätsindex des Polymeren berechnet als den Quotienten aus dem Gewichtsmittel und dem Zahlenmittel nach GPC. Die Polymerisationen wurden bis zu einem Umsatz von 60 % geführt.

**Tabelle 3**

| Regler-Verbindung | Variante | Tl. Regler/$M_n$(ber.) | $M_n$ (GPC) | Mw/Mn | Beispiel |
|---|---|---|---|---|---|
| (1) | A | 0.35T1. / 40.000 | 49.000 | 1.5 | 7 |
| (1) | A | 0.116T1. / 120.000 | 130.000 | 1.8 | 8 |
| (1) | B | 0.116T1. / 120.000 | 104.000 | 1.7 | 9 |
| (2) | A | 0.233T1. / 60.000 | 115.000 | 2.0 | 10 |
| (1) | A* | 0.233T1. / 60.000 | 74.000 | 1.6 | 11 |

(fortgesetzt)

| Regler-Verbindung | Variante | Tl. Regler/$M_n$(ber.) | $M_n$ (GPC) | Mw/Mn | Beispiel |
|---|---|---|---|---|---|
| (1) | A** | 0.233T1. / 60.000 | ---*** | --- | 12 |
| * Monomereinsatz: 94 Teile (Tl.) Chloropren und 6 Teile 2,3-Dichlorbutadien<br>** Monomer: 2,3-Dichlorbutadien<br>*** GPC-Messung nicht möglich; Viskositätsmessungen der Umsatzproben zeigen Anstieg im Molekulargewicht | | | | | |

Aus den Ergebnissen der Tabellen 1-3 können folgende Schlüsse abgeleitet werden:

- Die getesteten Xanthogensäureester sind als Regler für die Polymerisation von Chloropren unter den gewünschten Bedingungen ungeeignet.

- Nur Dithiocarbaminsäureester basierend auf sekundären Aminen mit einem pKs der protonierten Form zwischen 12 und 20, bevorzugt 14-18 zeigen eine Aktivität als Regler bei der Emulsionspolymerisation von Chloropren und 2,3-Dichlorbutadien. Die Aktivität ist deutlich höher als die bekannter Regler auf Basis von Xanthogendisulfiden (DE-A 304 48 11).

- Von den getesteten Dithiocarbaminsäureestern zeichnen sich besonders die erfindungsgemäßen Verbindungen (1) und (2) aus. Mit diesen Verbindungen wird eine gute Übereinstimmung des Umsatzes mit dem Molekulargewicht nach Gleichung 1 erreicht.

- Die Aktivität der erfindungsgemäßen Regler ist im gesamten bei der Emulsionspolymerisation von Chloropren relevanten Temperaturbereich von 5 bis 80°C, bevorzugt 10 bis 45°C und im Zusammenspiel mit allen für Emulsionspolymerisation von Chloropren gängigen Initiatorsystemen sehr gut.

Die notwendige Polymerisationszeit von 1 bis 5 Stunden zur Erreichung eines Umsatzes von 60% gewährleistet die Möglichkeit der Wärmeabfuhr auch in der großtechnischen Produktion.

**Beispiele zum Verfahren zur erfindungsgemäßen Darstellung von Dithiocarbaminsäureestern:**

Zur Darstellung von Dithiocarbaminsäureestern nach dem erfindungsgemäßen Verfahren wurden 500 ml THF (wasserfrei) und 1 mol metallisches Kalium (Variante A) oder flüssige NaK Legierung (Variante B) oder Kaliumhydrid (Variante C) in einem 1 L Vierhalskolben mit Rührer, Thermometer und Tropftrichter unter Stickstoffatmosphäre bei Raumtemperatur vorgelegt. Dazu wurden 1 mol Aminverbindung (gelöst in 150 ml THF) innerhalb von 0,5 h zugetropft. Nach vollständigem Umsatz des Alkalimetalls mit dem Amin zum Salz (Sichtkontrolle), wurde 1 mol Schwefelkohlenstoff (gelöst in 240 ml THF) zugegeben und 1h gerührt. Das entstandene Dithiocarbamatsalz wurde ohne vorherige Isolation des Carbaminsäuresalzes mit 1,5 mol Organohalogenidverbindung zum gewünschten Dithiocarbaminsäureester umgesetzt. Die Reaktion erfolgte unter GC-Kontrolle (GC = Gaschromatographie) bis zum vollständigen Umsatz des Dithiocarbaminsäuresalzes. Zur Abtrennung des entstandenen Alkalihalogenids wurde das Lösungsmittel im Vakuum entfernt und der Rückstand mit 500mL Pentan aufgeschlämmt. Nach Abfiltrieren des Alkalihalogenids wurde das Produkt nach Einengen der Lösung im Vakuum von 0,1 bar bei 50°C in ausreichender Reinheit erhalten. Nicht umgesetztes Organohalogenid wurde dabei zurückgewonnen.

Führt man die gleichen Versuche durch, ersetzt das Alkalimetall Kalium jedoch gegen Natrium (Variante D) oder Natriumhydrid (Variante E), so erhält man nach dieser Reaktionsführung ein stark mit Nebenprodukten verunreinigtes Produkt, welches nur nach Anwendung fortgeschrittener Reinigungstechniken wie Chromatographie die notwendige Reinheit erhält, um in der Chloropren-Polymerisation adäquate Ergebnisse zu produzieren.

Tabelle 4 listet die Ergebnisse der Versuche zur Darstellung geeigneter Dithiocarbaminsäureester zur kontrollierten Polymerisation von Chloropen auf. Spalte 1 beinhaltet die Variante der Versuchsdurchführung. Spalte 2 und 3 zeigen die Komponenten Z und R der Reglerverbindung auf. In Spalte 4 wird die Ausbeute an Reglersubstanz in % aufgeführt. Spalte 5 differenziert zwischen erfindungsgemäßen Beispielen (Ziffern) und Vergleichsbeispielen (Buchstaben).

**Tabelle 4**

| Variante | Amin (Z) | Organohalogenid (R-X) | Ausbeute | Beispiele/ Vergleichs beispiele |
|---|---|---|---|---|
| A | Pyrrol | 1,3 Dichlor-2-buten | 81.5% | 13 |
| A | Imidazol | 1,3 Dichlor-2-buten | 80.6% | 14 |
| B | Pyrrol | 1,3 Dichlor-2-buten | 78.5% | 15 |
| c | Pyrrol | 1,3 Dichlor-2-buten | 65% | 16 |
| D | Pyrrol | 1,3 Dichlor-2-buten | 72%* | K |
| E | Pyrrol | 1,3 Dichlor-2-buten | 78%* | L |
| * Produktreinheit ca. 80 % | | | | |

**Vergleichsbeispiele (nach WO 99/31144)**

**M: Synthese von Benzyl-1-pyrrolcarbodithioat:**

Unter Rühren wurde zu einer Suspension von Natriumhydrid (0,48g, 20mmol) in Dimethylsulfoxid (20mL) Pyrrol (1,34g, 20mmol) zugetropft. Nach beendeter Zugabe wurde die resultierende braune Suspension für weitere 30 min. bei Raumtemperatur gerührt, bevor das Kohlenstoffdisulfid (1,52 g, 20 mmol) zugesetzt wurde. Die Lösung wurde weitere 30 min. bei Raumtemperatur gerührt und schließlich das Benzylchlorid (2,53 g, 20 mmol) zugegeben. Nach 1 Std. wurde Wasser (20 mL), gefolgt von Diethylether (20 mL), der Reaktionsmischung zugefügt. Nach Abtrennung der organischen Phase wurde die wässrige Phase zweimal mit je 20 mL Diethylether extrahiert. Die vereinten organischen Phasen wurden mit Magnesiumsulfat getrocknet, filtriert und das Lösungsmittel abdestilliert. Das Rohprodukt wurde zur Isolierung des Produktes chromatographisch mittels 5 %-igem Ethylacetat in Petrolether aufgetrennt. Das Reinprodukt wurde als gelbes Öl in 50 %-iger Ausbeute (2,34 g) isoliert.

**N: Synthese von Benzyl-1-imidazolcarbodithioat:**

Zu einer Lösung von Thiocarbonyldiimidazol (0,89 g, 5,5 mmol) in Dichlormethan (10 mL) wurde unter Rühren das Benzylmercaptan (0,68 g, 5 mmol) bei Raumtemperatur zugetropft. Die Lösung wurde bei gleicher Temperatur 30 min gerührt und das Lösungsmittel schließlich im Vakuum entfernt. Der Rückstand wurde chromatographisch (Kieselgel-60, 70-230 mesh) mit einem Lösungsmittelgemisch bestehend aus Ethylacetat und Petrolether im Verhältnis 3:7 als Eluent aufgetrennt. Dadurch konnte das Produkt Benzyl-1-imidazolcarbodithioat (65) als leicht gelber Feststoff in 54 %-iger Ausbeute (0,78 g) isoliert werden.

Nach direktem Vergleich des erfindungsgemäßen Verfahrens mit literaturbekannten und oben zitierten Verfahren zeigt das erfindungsgemäße Verfahren deutliche Vorteile in der Produktausbeute sowie insbesondere in dem Wegfall aufwendiger und ausbeutemindernder Reinigungsschritte.

Nach diesem Verfahren dargestellte, erfindungsgemäße Regler sind in der Lage die Emulsionspolymerisation von Chloropren und 2,3-Dichlorbutadien sowie die Copolymerisation von Chloropren und 2,3-Dichlorbutadien mit Vinyl- und Dienmonomeren bezüglich des Molekulargewichts und der Mikrostruktur (Copolymerzusammensetzung, Endgruppen) zu steuern.

**Patentansprüche**

**1.** Eine Verbindung der Formel (I),

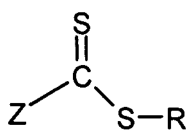

(I)

worin

R ein halogensubstituierter Alkenylrest ist,

und worin

Z auf einer der folgenden Bedeutungen basiert: Pyrrol, Imidazol, Pyrazol, Indol, Carbazol, 2-Piperidinon, 2-Azepanon, 2-Azocanon.

**2.** Ein Verfahren zur Herstellung der Verbindung der Formel (I) nach Anspruch 1 umfassend

a) das Bereitstellen einer Verbindung der Formel (IV),

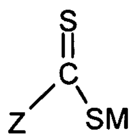

(IV)

wobei Z die in Anspruch 1 genannte Bedeutung hat und M Kalium ist,
b) das Umsetzen dieser Verbindung mit einer Verbindung der Formel (V)

R-X (V)

wobei R die in Anspruch 1 genannte Bedeutung hat und X für Cl, Br oder I steht.

**3.** Verwendung der Verbindung der Formel (I) wie definiert in Anspruch 1 zur Regelung des Polymerisationsgrades bei der Polymerisation von Monomeren wobei zumindest ein Monomer oder alle eine Diengruppe enthalten, wobei die Polymersation in Emulsion durchgeführt wird.

**4.** Die Verwendung nach Anspruch 3, wobei die Monomere Chloropren und/oder 2,3-Dichlorbutadien sind.

**5.** Ein Verfahren zur Herstellung eines Polymeren durch Polymerisation von Monomeren, wobei zumindest ein Monomer oder alle eine Diengruppe enthalten, in Gegenwart der Verbindung der Formel (I) wie definiert in Anspruch 1, wobei die Herstellung der Polymeren in Emulsion durchgeführt wird.

**6.** Das Verfahren nach Anspruch 5, wobei die Monomere Chloropren und/oder 2,3-Dichlorbutadien sind.

**7.** Ein Polymer, hergestellt durch Polymerisation von Monomeren, wobei zumindest ein Monomer oder alle Monomere eine Diengruppe enthalten, in Gegenwart der Verbindung der Formel (I), wie in Anspruch 1 definiert, **dadurch gekennzeichnet, dass** das Polymer Endgruppen der Formel (VI)

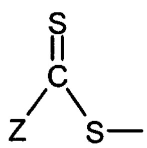

(VI)

und Endgruppen der Formel (VI a)

R - (VIa)

enthält,
wobei Z und R die in Anspruch 1 definierte Bedeutung haben.

**8.** Das Polymer nach Anspruch 7, wobei das Polymer Polychloropren oder Poly-2,3-dichlorobutadien oder Polychloropren-co-2,3-dichlorobutadien ist.

## Claims

1. A compound of the formula (I),

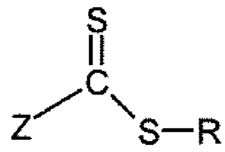

(I)

where

R is a halogen-substituted alkenyl radical,

and where

Z is based on one of the following: pyrrole, imidazole, pyrazole, indole, carbazole, 2-piperidinone, 2-azepanone, 2-azocanone.

2. A process for preparing the compound of the formula (I) according to Claim 1, comprising

a) providing a compound of the formula (IV),

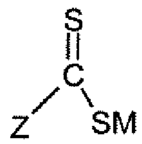

(IV)

where Z is as defined in Claim 1 and M is potassium,
b) reacting this compound with a compound of the formula (V)

R-X (V),

where R is as defined in Claim 1 and X is Cl, Br or I.

3. Use of the compound of the formula (I) as defined in Claim 1 for regulating the degree of polymerization during the polymerization of monomers, where at least one monomer or all contain a diene group, where the polymerization is carried out in emulsion.

4. The use according to Claim 3, where the monomers are chloroprene and/or 2,3 dichlorobutadiene.

5. A process for preparing a polymer by polymerizing monomers, where at least one monomer or all contain a diene group, in the presence of the compound of the formula (I) as defined in Claim 1, where the preparation of the polymers is carried out in emulsion.

6. The process according to Claim 5, where the monomers are chloroprene and/or 2,3-dichlorobutadiene.

7. A polymer prepared by polymerization of monomers, where at least one monomer or all monomers contain a diene group, in the presence of the compound of the formula (I), as defined in Claim 1, **characterized in that** the polymer contains end groups of the formula (VI)

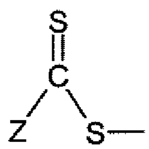

(VI)

and end groups of the formula (VI a)

R - (VIa),

where Z and R are as defined in Claim 1.

**8.** The polymer according to Claim 7, where the polymer is polychloroprene or poly-2,3-dichlorobutadiene or polychloroprene-co-2,3-dichlorobutadiene.

## Revendications

**1.** Composé de formule (I)

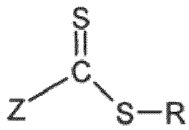

(I)

dans laquelle

R est un radical alcényle à substitution halogène, et
Z est à base d'une des significations suivantes : pyrrole, imidazole, pyrazole, indole, carbazole, 2-pipéridinone, 2-azépanone, 2-azocanone.

**2.** Procédé de fabrication du composé de formule (I) selon la revendication 1, comprenant :

a) la préparation d'un composé de formule (IV)

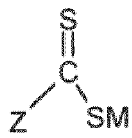

(IV)

dans laquelle Z a la signification indiquée dans la revendication 1 et M représente le potassium,
b) la mise en réaction de ce composé avec un composé de formule (V)

R-X (V)

dans laquelle R a la signification indiquée dans la revendication 1 et X représente Cl, Br ou I.

**3.** Utilisation du composé de formule (I) tel que défini dans la revendication 1 pour la régulation du degré de polymérisation lors de la polymérisation de monomères, au moins un monomère ou tous contenant un groupe diène, la polymérisation étant réalisée en émulsion.

**4.** Utilisation selon la revendication 3, dans laquelle les monomères sont le chloroprène et/ou le 2,3-dichlorobutadiène.

**5.** Procédé de fabrication d'un polymère par polymérisation de monomères, au moins un monomère ou tous contenant un groupe diène, en présence du composé de formule (I) tel que défini dans la revendication 1, la fabrication des polymères étant réalisée en émulsion.

**6.** Procédé selon la revendication 5, dans lequel les monomères sont le chloroprène et/ou le 2,3-dichlorobutadiène.

**7.** Polymère, fabriqué par polymérisation de monomères, au moins un monomère ou tous les monomères contenant un groupe diène, en présence du composé de formule (I) tel que défini dans la revendication 1, **caractérisé en ce**

**que** le polymère contient des groupes terminaux de formule (VI)

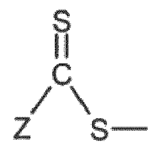

(VI)

et des groupes terminaux de formule (VIa)

R - (VIa)

Z et R ayant la signification définie dans la revendication 1.

**8.** Polymère selon la revendication 7, dans lequel le polymère est le polychloroprène ou le poly-2,3-dichlorobutadiène ou le polychloroprène-co-2,3-dichlorobutadiène.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 9931144 A
- DE 2156453 A
- WO 9801478 A
- WO 0142312 A
- WO 9858974 A
- WO 9935177 A
- CH 533106 A
- DE 2131135 A
- DE 111804 A1
- DE 1200988 A
- DE 3044811 A


### In der Beschreibung aufgeführte Nicht-Patentliteratur


- *Tetrahedron Letters,* 1999, 2435 ff
- Houben-Weyl. Thieme Verlag, 1983, vol. 4, 458-478
- *Journal of Heterocyclic Chemistry,* 1994, vol. 31 (4), 997-1004
- *Synthesis,* 1978, 370-372